(19) Europäisches Patentamt / European Patent Office / Office européen des brevets

(11) **EP 1 945 738 B1**

(12) **EUROPÄISCHE PATENTSCHRIFT**

(45) Veröffentlichungstag und Bekanntmachung des Hinweises auf die Patenterteilung:
**08.08.2018 Patentblatt 2018/32**

(21) Anmeldenummer: **06818548.7**

(22) Anmeldetag: **15.11.2006**

(51) Int Cl.:
***C10G 19/02*** *(2006.01)*

(86) Internationale Anmeldenummer:
**PCT/EP2006/010946**

(87) Internationale Veröffentlichungsnummer:
**WO 2007/057165 (24.05.2007 Gazette 2007/21)**

(54) **VERFAHREN ZUR GERUCHSENTFERNUNG BEI FLÜSSIGEN KOHLENWASSERSTOFFEN**

PROCESS FOR DEODORISING LIQUID HYDROCARBONS

PROCÉDÉ DE DÉSODORISATION D'HYDROCARBURES LIQUIDES

(84) Benannte Vertragsstaaten:
**DE ES FR GB IT**

(30) Priorität: **16.11.2005 EP 05024998**

(43) Veröffentlichungstag der Anmeldung:
**23.07.2008 Patentblatt 2008/30**

(73) Patentinhaber: **Cognis IP Management GmbH**
**40589 Dusseldorf (DE)**

(72) Erfinder:
• **DIERKER, Markus**
**40593 Düsseldorf (DE)**
• **FALKOWSKI, Jürgen**
**40789 Monheim (DE)**

(74) Vertreter: **BASF IP Association**
**BASF SE**
**G-FLP-C006**
**67056 Ludwigshafen (DE)**

(56) Entgegenhaltungen:
DE-C- 925 059        US-A- 2 874 115
US-A- 2 883 338      US-A- 3 818 105
US-A1- 2003 052 046

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Entfernung von unerwünschten Gerüchen bei flüssigen Kohlenwasserstoffen. Leichtflüchtige Ölkörper, sog. 'light emollients' werden von der kosmetischen Industrie in einer Vielzahl von Formulierungen verwendet. Insbesondere für die dekorative Kosmetik bzw. in pflegenden Formulierungen werden große Mengen an leichterflüchtigen Komponenten eingesetzt. Bei diesen Komponenten kann es sich beispielsweise um flüchtige, cyclische Silikone (z.B: Cyclopentasiloxan oder Cyclomethicon) oder Kohlenwasserstoffe aus petrochemischen Prozessen handeln. Bei den zuletzt genannten Stoffen handelt es sich aufgrund ihrer Herstellung überwiegend um Gemische aus linearen und verzweigten Kohlenwasserstoffen. Beispiele und anwendungstechnische Beschreibungen derartiger Formulierungen können in Standardwerken, wie zum Beispiel: 'Handbook of Cosmetic Science and Technology', A. Barel, M. Paye, H. Maibach, Marcel Dekker Inc. 2001 nachgelesen werden. Alle genannten Rohstoffe müssen die hohen Qualitätsanforderungen in kosmetischen Formulierungen erfüllen. Neben der toxikologischen Unbedenklichkeit dürfen diese Rohstoffe auch keine Restmengen an qualitätsmindernden Komponenten enthalten, die beispielsweise zu einer geruchlichen Beeinträchtigung der kosmetischen Formulierung führt.

Aufgabe dieser Erfindung bestand darin leichtflüchtige Ölkörper zu finden, die einerseits toxikologisch unbedenklich sind und andererseits in typischen kosmetischen Formulierungen ohne anwendungstechnische Einschränkungen eingesetzt werden können. Einfache lineare und gesättigte Kohlenwasserstoffe, die beispielsweise durch Hydrierung von Olefinen gewonnen werden können, erfüllen in vielen Fällen dieses Anforderungsprofil. Allerdings stellt man fest, dass diese Rohstoffe nach einem Destillationsschritt zur Erzielung der gewünschten Eigenschaften bzgl. Reinheit und Flüchtigkeit einen in kosmetischen Formulierungen nicht akzeptablen Aufgeruch aufweisen.

Reinste lineare Kohlenwasserstoffe, die bei Raumtemperatur noch flüssig sind und mit sehr aufwendigen Laborverfahren, wie beispielsweise chromatographischen Trennverfahren, aufgereinigt worden sind, sind praktisch geruchsfrei. Der Aufgeruch der destillierten Kohlenwasserstoffe lässt sich auch nicht durch einen Desodorierungsschritt, der nach technisch bekannten Methoden mit Inertgasen wie Wasserdampf oder Stickstoff durchgeführt, beseitigen. Die Ursache dieses Aufgeruches sind wahrscheinlich nicht quantifizierbare Verunreinigungen, die während der komplexen Herstellung entstehen. Bei den Herstellprozessen für unverzweigte höhere Olefine handelt es sich meistens um Oligomerisierungen von niederen Kohlenwasserstoffen, wie beispielsweise die Oligomerisierung von Ethen in Aufbaureaktionen zu den sog. Ziegler-Olefinen oder Verfahren mit metallorganischen Mischkatalysatoren wie im SHOP-Prozess der Fa. Shell. Verzweigte höhere Olefine werden bevorzugt durch Oligomerisierung bzw. Co-oligomerisierung niederer Olefine, wie Propen, iso-Buten und n-Buten, hergestellt, wobei überwiegend saure Katalysatoren wie beispielsweise beim Bayer-Verfahren für iso-Buten oder metallorganische Katalysatoren verwendet werden.

Es wurde nun überraschend gefunden, dass Kohlenwasserstoffe durch ein einfaches Verfahren von den störenden Gerüchen befreit werden können.

Ein erster Gegenstand der vorliegenden Anmeldung betrifft daher ein Verfahren zur Geruchsentfernung bei flüssigen Kohlenwasserstoffen, wobei die flüssigen Kohlenwasserstoffe mit einer wässerigen Flüssigkeit mit einem pH-Wert > 7 in Kontakt gebracht und gerührt werden, wobei es wesentlich ist, dass falls das Verfahren als diskontinuierliches Verfahren durchgeführt wird der mechanische Leistungseintrag in das System aus den flüssigen Kohlenwasserstoffen und der wässerigen Flüssigkeit zwischen 2 und 200 W/kg beträgt, oder falls das Verfahren als kontinuierliches Verfahren durchgeführt wird, der mechanische Energieeintrag in das System aus den flüssigen Kohlenwasserstoffen und der wässerigen Flüssigkeit mindestens

1 kJ/Kg und höchstens 100 kJ/kg beträgt. Das vorliegende Verfahren eignet sich nur für Kohlenwasserstoffe, die flüssig vorliegen, vorzugsweise aber für solche Kohlenwasserstoffe, die bei Raumtemperatur also 21 °C bereits flüssig sind. Das Verfahren kann aber auch bei höheren Temperaturen Anwendung finden, z.B. wenn höher schmelzende Kohlenwasserstoffe zu reinigen sind.

Unter Kohlenwasserstoffen werden im Folgenden Alkane und Alken und zwar sowohl lineare wie verzweigte Isomere, aber auch ringgeschlossene Kohlenwasserstoffe und deren beliebigen Mischungen untereinander verstanden.

Dass Kohlenwasserstoffe mit Alkalilaugen in Kontakt gebracht werden und so eine Reinigung möglich ist, ist an sich bereits bekannt. In der US 1,553,141 wird ein Verfahren beschrieben, bei dem aus natürlichen oder synthetischen Ölen Verunreinigungen durch das Vermischen mit einer alkalischen wässerigen Lösung entfernt werden können. Das Dokument gibt aber weder einen Hinweis auf die Möglichkeit, so den Geruch der Öle zu verbessern noch werden Angaben über den Energieeintrag gemacht. Des Weiteren betrifft die US 1,553,141 keine Kohlenwasserstoffmischungen für den Einsatz in kosmetischen Mitteln.

Die US 1,951,324 beschreibt ein Verfahren zur Geruchsentfernung für Kohlenwasserstoffe, wobei die Kohlenwasserstoffe mit einer wässerigen Bleioxid-Lösung in Kontakt gebracht werden und durch nachfolgende Zugabe von Schwefel das Blei als Sulfid ausgefällt werden muss.

Es ist aber von entscheidender Bedeutung bei der Durchführung des vorliegenden erfindungsgemäßen Verfahrens, dass die Kohlenwasserstoff- oder Ölphase mit der wässrigen Alkaliphase intensiv vermischt wird, d.h. eine ausreichende Phasengrenzfläche zwischen diesen beiden ineinander unlöslichen Phasen gebildet wird. Zur Erzielung dieser Phasengrenzfläche muss eine ausreichende Menge an mechanischer Energie in das Flüssig/Flüssig-System eingebracht werden.

Diskontinuierliche Verfahren sind dadurch charakterisiert, dass der Energieeintrag auf das gesamte Reaktionsgemisch erfolgt, üblicherweise können hierzu bei diskontinuierlichen Prozessen in Rührkesseln spezielle Dispergierrührwerke verwendet werden. Der Leistungsbedarf in [W] für ein gerührtes System kann beispielsweise nach folgender Formel errechnet werden:

$$P = Ne * \rho * n^3 * d^5$$

wobei Ne die von der Rührergeometrie und Reynoldszahl abhängige Newtonzahl, p die mittlere Dichte in [kg/m$^3$] des gerührten Stoffsystems, n die Rührerdrehzahl in [1/s] und d der Rührerdurchmesser in [m] ist.

Um den Energieeintrag bei kontinuierlich bzw. diskontinuierlich betriebenen Prozessen mit verschiedenen Dispergiersystemen vergleichen zu können definiert man einen spezifischen Energieeintrag Q in [J/kg], der den Energieeintrag pro Masseeinineinheit beschreibt.

Für diskontinuierlich betriebene Verfahren gemäß der Erfindung ist ein mechanischer Leistungseintrag von mindestens 2, insbesondere mindestens 5, vorzugsweise mindestens 10 W/kg erforderlich. Bevorzugt ist ein mechanischer Leistungseintrag im Bereich von 2 bis 200, insbesondere im Bereich von 5 bis 100 W/kg.. Dieser spezifische Leistungseintrag Ps kann für ein diskontinuierliches Verfahren durch Energieeintrag pro Rührzeit berechnet werden:

$$Ps\ [W/kg] =\ \text{spezifischen Energieeintrag Q [J/kg] / Rührzeit [s]}$$

Aus der oben genannten Formel ergibt sich somit für ein diskontinuierlich gerührtes System als Energieeintrag:

$$Q = (P * t)/m$$

Wobei t die Rührzeit in [s] und m die Masse des gerührten Stoffsystems in [kg] ist. Um den erfindungsgemäßen spezifischen Leistungseintrag bei diskontinuierlichen Verfahren zu erzielen, können Energieeintrag sowie Rührzeiten variiert werden. Übliche Energieeinträge können hierbei beispielsweise zwischen 1 bis 100 kJ/kg gewählt werden. Die Zeiten, in denen die beiden Phasen miteinander in Kontakt gebracht werden, wählt der Fachmann dann in Abhängigkeit des gewünschten spezifischen Leistungseintrages. Diese Zeiten können entsprechend variieren, wobei Zeiten von 1 bis 300 Minuten, vorzugsweise 1 bis 60 Minuten oder 1 bis 30 Minuten bevorzugt seien können. Umgekehrt kann der Fachmann bei vorgegebenen Rührzeiten den zur Erzielung des erforderlichen spezifischen Leistungseintrages erforderlichen Energieeintrag wählen.

Bei herkömmlichen, nicht-erfindungsgemäßen Rührverfahren liegt der spezifische mechanische Leistungseintrag für niedrigviskose Systeme üblicherweise bei 0,1 bis 1 W/kg.

Kontinuierliche Verfahren sind dadurch charakterisiert, dass der Energieeintrag kontinuierlich auf einen Teil des gesamten Reaktionsgemisches erfolgt, üblicherweise können bei kontinuierlichen Prozessen Zahnkranzdispergiermaschinen, Kolloidmühlen oder Hochdruckhomogenisatoren verwendet werden. Für ein kontinuierliches System errechnet sich der Energieeintrag Q analog zu

$$Q = P/ms$$

wobei ms der Massenstrom des Zweiphasensystems in [kg/s] ist. Wird das erfindungsgemäße Verfahren als kontinuierliches Verfahren durchgeführt, ist ein mechanischer Energieeintrag von mindestens 1 kJ/kg, vorzugsweise von mindestens 2 kJ/kg, insbesondere von mindestens 5 kJ/kg für das Zweiphasensystems erforderlich. Vorzugsweise liegt der mechanische Energieeintrag zwischen 1 und 100, vorzugsweise 2 bis 70, insbesondere zwischen 5 bis 60 und insbesondere zwischen 5 bis 45 kJ/kg. Im Gegensatz dazu wird bei herkömmlichen, nicht erfindungsgemäßen, diskontinuierlichen Verfahren mit deutlich weniger Energieeintrag gearbeitet, typisch sind Werte von unter 1 kJ/kg.

Ein erheblich größerer spezifischer Leistungseintrag (diskontinuierliche Verfahren) bzw. ein erheblich größerer Energieeintrag (kontinuierliche Verfahren) unter den oben erläuterten Temperatur- und Konzentrationsgrenzen ist zwar möglich, da der zu reinigende Kohlenwasserstoff unter diesen Prozessbedingungen praktisch chemisch inert ist, führt aber zu einem unwirtschaftlich hohen Aufwand an Energie- bzw. Apparatekosten. Es ist daher bevorzugt den maximalen spezifischen Leistungseintrag bei diskontinuierlichen Verfahren auf 200 W/kg und den maximale Energieeintrag bei kontinuierlichen Verfahren auf 100 kJ/kg zu beschränken.

Nach der erfolgten Alkalibehandlung erfolgt eine Phasentrennung und die Kohlenwasserstoffphase kann anschließend - unabhängig davon, ob das Verfahren als diskontinuierliches Verfahren oder als kontinuierliches Verfahren durchgeführt wurde - beispielsweise durch Zugabe von vollentsalztem Wasser und anschließender Phasentrennung - der Kohlenwasserstoff von der restlichen Menge an Alkalilösungen befreit werden. Es ist bevorzugt, dass die Kohlenwasserstoffphase im erfindungsgemäßen Verfahren nach dem Abtrennen der wässerigen Phase durch Destillation gereinigt wird.

Das erfindungsgemäße Verfahren wird vorzugsweise unter Verwendung von verdünnten Alkalilaugen durchgeführt. Hierbei können prinzipiell alle Alkalilaugen verwendet werden, die mindestens ein Kation aus der Gruppe der Alkali- bzw. Erdalkalimetalle enthalten, wobei vorzugsweise Natron- bzw. Kalilauge verwendet wird. Bleioxid oder andere wasserlösliche Bleiverbindungen sind vom Schutz ausgenommen.

Diese Laugen können in einem Konzentrationsbereich von 0,1% bis zur Löslichkeitsgrenze des entsprechenden Alkali- bzw. Erdalkalihydroxides in Wasser verwendet werden. Der bevorzugte Konzentrationsbereich liegt zwischen 2 bis 60 Gew.-%, vorzugsweise zwischen 3 bis 50 Gew.-%, wobei insbesondere der Bereich von 5 bis 20 Gew.-% angewendet wird. Anstelle von einfachen Alkalilösungen können auch Lösungen von Metallhydriden, wie beispielsweise Natriumborhydrid oder Lithiumaluminiumhydrid verwendet werden. Insbesondere eine technisch übliche Lösung von 12 Gew.-% Lithiumaluminiumhydrid und 40 Gew.-% Natriumhydroxid in 48 Gew.-% vollentsalztem Wasser, die unter dem Handelsnamen *Venpure®Solution* bekannt ist, kann mit den oben beschriebenen Einsatzkonzentrationen für das erfindungsgemäße Verfahren verwendet werden.

Die Behandlung mit der verdünnten Alkalilösung kann prinzipiell in einem Temperaturbereich von 0°C bis 250 °C erfolgen, vorzugsweise von 15 °C bis 150 °C und insbesondere von 20 bis 100 °C, wobei insbesondere in einem technischen Reaktor ein Bereich von 20°C bis 100 °C mit Vorteil angewendet wird. Der ganz besonders bevorzugte Temperaturbereich liegt zwischen 40 °C und 80 °C.

Bei den Kohlenwasserstoffen kann es sich um ungesättigte oder vorzugsweise um gesättigte Kohlenwasserstoffe handeln, die durch Hydrierung aus den entsprechenden ungesättigten Verbindungen hergestellt worden sind. Die Kohlenwasserstoffe können von linearer, verzweigter oder zyklischer Struktur sein, wobei es sich auch um physikalische Mischungen von linearen, verzweigten bzw. zyklischen Kohlenwasserstoffen handeln kann. In der molekularen Struktur der gesättigten oder ungesättigten Kohlenwasserstoffe können auch lineare, verzweigte und zyklische Strukturen gemeinsam in beliebiger Kombination vorhanden sein, also beispielsweise ein gesättigter Kohlenwasserstoffring mit einem ungesättigten linearen Substituenten. Bei dem erfindungsgemäß beschriebenen Verfahren kann es sich um Kohlenstoffverbindungen mit 6 bis 30 Kohlenstoffatomen handeln, wobei der bevorzugte Bereich bei 8 bis 20 Kohlenstoffatomen liegt. Insbesondere werden alle bei Raumtemperatur flüssigen Kohlenwasserstoffe für das erfindungsgemäße Verfahren bevorzugt.

Es ist weiterhin bevorzugt, dass das Mengenverhältnis zwischen den Kohlenwasserstoffen und der alkalischen wässerigen Flüssigkeit im Bereich von 100 : 1 bis 10 : 1 liegt.

Die Erfindung hat folgende Vorteile:

- Kohlenwasserstoffe können als preiswerte Rohstoffquelle zur Herstellung von leichtflüchtigen Ölkomponenten für kosmetische Anwendungen verwendet werden.
- Das erfindungsgemäß beschriebene Aufarbeitungsverfahren ist mit geringem technischen Mehraufwand zu realisieren
- Die Produktverluste sind im Vergleich zu einer Desodorierung von leichtflüchtigen Produkten niedriger.
- Die Langzeitstabilität der Produkte bzgl. des Geruchs ist deutlich besser als bei desodorierten Produkten, da die nicht bekannten bzw. nicht quantifizierbaren Geruchsträger effektiv entfernt werden können

Die nach dem erfindungsgemäßen Verfahren vom Geruch befreiten Kohlenwasserstoffe können in kosmetischen Zubereitungen und vorzugsweise zur Herstellung stabiler kosmetischer Emulsionen verwendet werden.

Vorzugsweise handelt es sich hierbei um Formulierungen zur Körperpflege, z. B. in Form von Cremes, Milch, Lotionen, sprühbaren Emulsionen, Produkten zur Eliminierung des Körpergeruchs etc. Die erfindungsgemäß gereinigten Kohlenwasserstoffen lassen sich auch in tensidhaltigen Formulierungen wie z. B. Schaum- und Duschbädern, Haarshampoos und Pflegespülungen einsetzen. Die kosmetischen Mittel können in Form von Emulsionen oder Dispersionen vorliegen, die Wasser und Ölphase nebeneinander enthalten. Bevorzugte kosmetische Zusammensetzungen sind solche in Form einer W/O- oder O/W-Emulsion mit den üblichen, dem Fachmann geläufigen, Konzentrationen an Ölen/ Fetten/Wachsen, Emulgatoren, Wasser und den in der Kosmetik üblichen, weiteren Hilfs- und Zusatzstoffen.

Typischerweise enthalten solche kosmetische Zusammensetzung 1 bis 50 Gew.-%, bevorzugt 5 bis 40 Gew.-% und insbesondere 5 bis 25 Gew.-%, Ölkörper, die zusammen mit z.B. öllöslichen Tensiden/Emulgatoren und öllöslichen Wirkstoffen Bestandteil der so genannten Öl- oder Fettphase sind. Zu den Ölkörpern werden Fettstoffe, Wachse, und flüssige Öle wie z.B. Kohlenwasserstoffe gerechnet, nicht aber Emulgatoren/Tenside. Die Kohlenwasserstoffe können als einziger Ölkörper oder aber in Kombination mit anderen Ölen/Fetten/Wachsen enthalten sein. Bevorzugt liegt der Anteil wenigstens eines Kohlenwasserstoffs bezogen auf die Gesamtmenge der Ölkörper bei 0,1 bis 100 Gew.-% und bevorzugt bei 1 bis 50 Gew.-%. Mengen von 1 bis 20 Gew.-% und insbesondere 3 bis 20 Gew.-% sind besonders bevorzugt.

Je nach Applikationszweck enthalten die kosmetischen Formulierungen eine Reihe weiterer Hilfs- und Zusatzstoffe wie beispielsweise oberflächenaktive Substanzen (Tenside, Emulgatoren), weitere Ölkörper, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfumöle, Farbstoffe etc.. Die Mengen der jeweiligen Zusätze richten sich nach der beabsichtigten Verwendung. Typische kosmetische Zusammensetzung enthalten zwischen 0,1 bis 20 Gew.-%, vorzugsweise 1 - 15 Gew.-% und insbesondere 1 - 10 Gew.-% einer oberflächenaktiven Substanz oder eines Gemisches aus oberflächenaktiven Substanzen.

### Beispiele

#### 1) Aufreinigungsversuch von Dodecan mit hohem Energieeintrag - diskontinuierliches Verfahren

1 kg Reaktionsprodukt Dodecan mit einer Jodzahl (JZ) von 0,04, welches über Hydrierung aus Dodecen hergestellt wurde, wird nach Filtration des Hydrierkatalysators in einem Laborrührkessel vorgelegt und auf 60°C aufgeheizt. Anschließend werden 0,5 kg einer 10%igen Natronlauge zugegeben und das ganze Zweiphasensystem unter Rühren auf 60°C aufgeheizt. Danach wird 5 min mit einem Ultra-Turrax (Bauart: T 50 der Fa. IKA ; Max. Leistung: 1,1 kW bei 10.000 U/min), der mit einer Dispergierscheibe als Rührwerkzeug ausgestattet war, mit einer Drehzahl von 6.000 U/min gerührt. Dies entspricht einem spezifischen Leistungseintrag von ca.160 W/kg und einem Energieeintrag von ca. 47 kJ/kg. Nach Phasentrennung wurde die Oberphase mit 0,5 kg vollentsalztem Wasser gewaschen und im Vakuum getrocknet. Der so hergestellte Kohlenwasserstoff konnte ohne Einschränkungen bzgl. störender Aufgerüche für kosmetische Formulierungen eingesetzt werden.

#### 2) Aufreinigungsversuch von Dodecan mit geringem Energieeintrag - diskontinuierliches Verfahren

Die gleiche Menge an Kohlenwasserstoff und die gleichen Einsatzmengen an Alkali wie in Beispiel 1 wurden bei 60°C mit einem herkömmlichen Laborrührwerk (Fa. IKA, Bauart: RW 20 DZM; Max. Leistung 70 W bei 500 U/min) für 30 min bei einer Drehzahl von 100 U/min verrührt. Dies entspricht einem spezifischen Leistungseintrag von 0,4 W/kg und einem Energieeintrag von ca. 0,7 kJ/kg.

Einen ähnlichen Energieeintrag würde man auch in einem Betriebsreaktor mit einer Einsatzmenge an Kohlenwasserstoff von beispielsweise 10.000 kg erreichen, falls man dieses System mit einer Rührwerksleistung von 5 kW ebenfalls für 30 min rührt wird. Nach Phasentrennung wurde die Oberphase mit 0,5 kg vollentsalztem Wasser gewaschen und im Vakuum getrocknet. Der so hergestellte Kohlenwasserstoff konnte bzgl. des störenden Aufgeruches nicht verbessert werden und war für kosmetische Formulierungen ungeeignet.

#### 3) Aufreinigungsversuch von Dodecan über Desodorierung

1 kg des im Beispiel 1 verwendeten Kohlenwasserstoffs wird in einen Laborrührkessel mit einer Verteilungseinrichtung für Inertgas vorgelegt und auf 80°C aufgeheizt. Anschließend wird zur Desodorierung bei einem Vakuum von 100 mbar für 1 h ein Stickstoffstrom von 1 Nm³/h durch den Kohlenwasserstoff durchgeleitet. Danach wird das Vakuum gebrochen, der Stickstoff abgedreht und das Produkt abgekühlt. Der so hergestellte Kohlenwasserstoff konnte bzgl. des störenden Aufgeruches nur geringfügig verbessert werden und war für kosmetische Formulierungen ungeeignet.

### Patentansprüche

1. Verfahren zur Geruchsentfernung bei flüssigen Kohlenwasserstoffen, wobei die flüssigen Kohlenwasserstoffe mit einer wässerigen Flüssigkeit mit einem pH-Wert > 7 in Kontakt gebracht und gerührt werden, **dadurch gekennzeichnet, dass** falls das Verfahren als diskontinuierliches Verfahren durchgeführt wird, der mechanische Leistungseintrag in das System aus den flüssigen Kohlenwasserstoffen und der wässerigen Flüssigkeit mindestens 2 W/kg beträgt oder falls das Verfahren als kontinuierliches Verfahren durchgeführt wird, der mechanische Energieeintrag in das System aus den flüssigen Kohlenwasserstoffen und der wässerigen Flüssigkeit mindestens 1 kJ/Kg beträgt, und anschließend die wässerige von der Ölphase getrennt wird.

**2.** Verfahren nach Anspruch 1, **dadurch gekennzeichnet, dass** der mechanische Leistungseintrag im Bereich von 2 bis 200 W/kg, vorzugsweise 5 bis 100 W/kg beträgt.

**3.** Verfahren nach Anspruch 1, **dadurch gekennzeichnet, dass** der mechanische Energieeintrag im Bereich von 1 bis 100, vorzugsweise 2 bis 70, vorzugsweise 5 bis 60 und insbesondere 5 bis 45 kJ/Kg beträgt.

**4.** Verfahren nach den Ansprüchen 1 bis 3, **dadurch gekennzeichnet, dass** es bei Temperaturen von O °C bis 250 °C, vorzugsweise von 15 °C bis 150 °C und insbesondere von 20 bis 100°C durchgeführt wird.

**5.** Verfahren nach den Ansprüchen 1 bis 4, **dadurch gekennzeichnet, dass** die wässerige Flüssigkeit Kationen aus der Gruppe der Alkali- und/oder Erdalkalimetalle enthalten.

**6.** Verfahren nach den Ansprüchen 1 bis 5, **dadurch gekennzeichnet, dass** als wässerige Flüssigkeit Lösungen von wasserlöslichen Salzen der Alkali- und/oder Erdalkalimetalle verwendet werden.

**7.** Verfahren nach den Ansprüchen 1 bis 6, **dadurch gekennzeichnet, dass** als wässerige Flüssigkeit eine Kalium- und/oder Natriumhydroxid-Lösung verwendet wird.

**8.** Verfahren nach den Ansprüchen 1 bis 7, **dadurch gekennzeichnet, dass** wässerige Lösungen von Metallhydriden, vorzugsweise von Natriumborhydrid oder Lithiumaluminiumhydrid verwendet werden.

**9.** Verfahren nach den Ansprüchen 1 bis 8, **dadurch gekennzeichnet, dass** die wässerige Flüssigkeit die alkalischen Verbindungen in Mengen von mindestens 0,1 Gew.-%, bezogen auf das Gewicht der wässerigen Flüssigkeit enthält.

**10.** Verfahren nach den Ansprüchen 1 bis 9, **dadurch gekennzeichnet, dass** als Kohlenwasserstoffe gesättigte, ungesättigte, lineare, verzweigte oder ringförmige Kohlenwasserstoffe mit 6 bis 30 Kohlenstoffatomen oder deren Mischungen ausgewählt sind.

**11.** Verfahren nach den Ansprüchen 1 bis 10, **dadurch gekennzeichnet, dass** als Kohlenwasserstoffe gesättigte Kohlenwasserstoffe mit 6 bis 30, vorzugsweise 10 bis 20 und insbesondere 12 bis 18 Kohlenstoffatomen, oder deren Mischungen ausgewählt sind.

**12.** Verfahren nach den Ansprüchen 1 bis 11, **dadurch gekennzeichnet, dass** die Mischung zwischen den Kohlenwasserstoffen und der wässerigen Flüssigkeit ein Mengenverhältnis (mim) von 100 : 1 bis 10 : 1 aufweist.

**13.** Verfahren nach den Ansprüchen 1 bis 12, **dadurch gekennzeichnet, dass** die Kohlenwasserstoffe nach der Behandlung mit der wässerigen Flüssigkeit destilliert werden.

**Claims**

**1.** A process for removal of odor in association with liquid hydrocarbons, the liquid hydrocarbons being contacted and stirred with an aqueous liquid having a pH > 7, wherein, if the process is carried out as a batch process, the mechanical power input into the system composed of the liquid hydrocarbons and the aqueous liquid is at least 2 W/kg, or, if the process is carried out as a continuous process, the mechanical energy input into the system composed of the liquid hydrocarbons and the aqueous liquid is at least 1 kJ/kg, and subsequently the aqueous phase is separated from the oil phase.

**2.** The process according to claim 1, wherein the mechanical power input is in the range from 2 to 200 W/kg, preferably 5 to 100 W/kg.

**3.** The process according to claim 1, wherein the mechanical energy input is in the range from 1 to 100, preferably 2 to 70, preferably 5 to 60 and more particularly 5 to 45 kJ/kg.

**4.** The process according to claims 1 to 3, which is carried out at temperatures from 0°C to 250°C, preferably from 15°C to 150°C and more particularly from 20 to 100°C.

**5.** The process according to claims 1 to 4, wherein the aqueous liquid comprises cations from the group of the alkali metals and/or alkaline earth metals.

6. The process according to claims 1 to 5, wherein use is made as aqueous liquid of solutions of watersoluble salts of the alkali metals and/or alkaline earth metals.

7. The process according to claims 1 to 6, wherein use is made as aqueous liquid of a potassium and/or sodium hydroxide solution.

8. The process according to claims 1 to 7, wherein aqueous solutions of metal hydrides, preferably of sodium borohydride or lithium aluminum hydride, are used.

9. The process according to claims 1 to 8, wherein the aqueous liquid comprises the alkaline compounds in amounts of at least 0.1% by weight, based on the weight of the aqueous liquid.

10. The process according to claims 1 to 9, wherein hydrocarbons selected are saturated, unsaturated, linear, branched or cyclic hydrocarbons having 6 to 30 carbon atoms, or mixtures thereof.

11. The process according to claims 1 to 10, wherein hydrocarbons selected are saturated hydrocarbons having 6 to 30, preferably 10 to 20 and more particularly 12 to 18 carbon atoms, or mixtures thereof.

12. The process according to claims 1 to 11, wherein the mixture of the hydrocarbons with the aqueous liquid has a quantitative ratio (m/m) of 100:1 to 10:1.

13. The process according to claims 1 to 12, wherein the hydrocarbons, after treatment with the aqueous liquid, are distilled.


**Revendications**

1. Procédé d'élimination des odeurs d'hydrocarbures liquides, selon lequel les hydrocarbures liquides sont mis en contact avec un liquide aqueux ayant un pH > 7 et agités, **caractérisé en ce que** si le procédé est réalisé sous la forme d'un procédé discontinu, l'apport de puissance mécanique dans le système constitué par les hydrocarbures liquides et le liquide aqueux est d'au moins 2 W/kg ou, si le procédé est réalisé sous la forme d'un procédé continu, l'apport d'énergie mécanique dans le système constitué par les hydrocarbures liquides et le liquide aqueux est d'au moins 1 kJ/kg, puis la phase aqueuse est séparée de la phase huileuse.

2. Procédé selon la revendication 1, **caractérisé en ce que** l'apport de puissance mécanique se situe dans la plage allant de 2 à 200 W/kg, de préférence de 5 à 100 W/kg.

3. Procédé selon la revendication 1, **caractérisé en ce que** l'apport d'énergie mécanique se situe dans la plage allant de 1 à 100, de préférence de 2 à 70, de préférence de 5 à 60 et notamment de 5 à 45 kJ/kg.

4. Procédé selon les revendications 1 à 3, **caractérisé en ce qu'**il est réalisé à des températures de 0 °C à 250 °C, de préférence de 15 °C à 150 °C, et notamment de 20 à 100 °C.

5. Procédé selon les revendications 1 à 4, **caractérisé en ce que** le liquide aqueux contient des cations du groupe des métaux alcalins et/ou alcalino-terreux.

6. Procédé selon les revendications 1 à 5, **caractérisé en ce que** des solutions de sels solubles dans l'eau des métaux alcalins et/ou alcalino-terreux sont utilisées en tant que liquide aqueux.

7. Procédé selon les revendications 1 à 6, **caractérisé en ce qu'**une solution d'hydroxyde de potassium et/ou de sodium est utilisée en tant que liquide aqueux.

8. Procédé selon les revendications 1 à 7, **caractérisé en ce que** des solutions aqueuses d'hydrures métalliques, de préférence de borohydrure de sodium ou d'hydrure de lithium et d'aluminium sont utilisées.

9. Procédé selon les revendications 1 à 8, **caractérisé en ce que** le liquide aqueux contient les composés alcalins

**EP 1 945 738 B1**

en quantités d'au moins 0,1 % en poids, par rapport au poids du liquide aqueux.

10. Procédé selon les revendications 1 à 9, **caractérisé en ce que** des hydrocarbures saturés, insaturés, linéaires, ramifiés ou cycliques contenant 6 à 30 atomes de carbone ou leurs mélanges sont choisis en tant qu'hydrocarbures.

11. Procédé selon les revendications 1 à 10, **caractérisé en ce que** des hydrocarbures saturés contenant 6 à 30, de préférence 10 à 20 et notamment 12 à 18 atomes de carbone, ou leurs mélanges sont choisis en tant qu'hydrocarbures.

12. Procédé selon les revendications 1 à 11, **caractérisé en ce que** le mélange des hydrocarbures et du liquide aqueux présente un rapport de quantité (mim) de 100:1 à 10:1.

13. Procédé selon les revendications 1 à 12, **caractérisé en ce que** les hydrocarbures sont distillés avec le liquide aqueux après le traitement.

8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 1553141 A

- US 1951324 A

**In der Beschreibung aufgeführte Nicht-Patentliteratur**

- Handbook of Cosmetic Science and Technology. Maibach, Marcel Dekker Inc, 2001